## Europäisches Patentamt

## European Patent Office

## Office européen des brevets

(11) Publication number: **0 231 536**
**A1**

## (12) EUROPEAN PATENT APPLICATION

(21) Application number: 86202068.2

(22) Date of filing: 04.03.83

(51) Int. Cl.4: **A61F 13/16** , A41B 13/02 ,
A61F 5/44

(30) Priority: 10.03.82 SE 8201489

(43) Date of publication of application:
**12.08.87 Bulletin 87/33**

(60) Publication number of the earlier application in
accordance with Art.76 EPC: **0 088 738**

(84) Designated Contracting States:
**BE DE FR GB LU NL**

(71) Applicant: **AB AKERLUND & RAUSING
Box 22
S-221 00 Lund(SE)**

(72) Inventor: **Fransson, Per
Trindyxvägen 9
S-291 65 Kristianstad(SE)**
Inventor: **Ekstrand, Stig
Envigsgränden 10
S-223 75 Lund(SE)**

(74) Representative: **Graudums, Valdis et al
Backers Patentbyra AB Drottninggatan 15
S-441 14 Göteborg(SE)**

(54) An Apparatus for manufacturing a fluid absorbing product.

(57) An apparatus for manufacturing products having
a fluid absorbing core and a fluid-impermeable layer
on a portion thereof.

A support web (II) is supplied from a supply
roller (I0) and means (I2, I5) are arranged for sup-
plying fluid absorbing material (I6) onto the web.
Furtheron, there are means (23, 26, 22) for folding
and sealing the support material around the core.

A suction device (I7) is periodically operable for
removing core material at locations where cross-wise
sealing will be carried out.

EP 0 231 536 A1

## AN APPARATUS FOR MANUFACTURING A FLUID ABSORBING PRODUCT

The present invention relates to a device for manufacturing a fluid absorbing product and is a divisional application of our EPC-application No. 83850051.0.

Known products of the actual type consist of high grade material and material combinations. In the most simple form thereof a fluid absorbing product may be regarded as a piece of equipment that allows inflow of a fluid to an absorbing core via a first side or portion thereof, but prevents penetration for the rest.

The inflow of a fluid for instance may take place through a layer of so called "fibre cloth" - (non-woven) which as such also might possess a certain absorption capacity. A high liquid absorption is obtained by cellulose based fibres - (cellulose, cotton, rayon), while synthetic fibres as polypropylene and polyester have a very low absorption capacity. As far as the pliability and handling of the non-woven material are concerned the fibre cloth material does not induce any problems in lines for manufacturing napkins, sanitary napkins, baby napkins, brief shields, sterile dressings, bandage material, etc. The material for instance may be manufactured with a surface weight as low as I0 g/m² and still have an acceptable strength.

In addition to the necessary characteristics of allowing passage of fluid to the absorbing core, the fibre cloth material also acts to maintain the core in a proper position. Generally, the core consists of defibrated cellulose (defibrate) and additionally might include inserts of "super absorbing" material.

In order to give the fluid absorbing product penetration preventing characteristics -a liquid leakage barrier -in the regions thereof where absorption is not desired, it previously simply has been inserted a loose film of plastics, for instance a polyethylene film, between the core and the carrier material. In certain cases the need for measures against leakage even has been neglected.

Another, more sophisticated technique using lamination and extrusion methods is disclosed in GB patent application No. 2 042 901.

According to the present invention it has been realized that there is a need for an efficient production machinery for completing the manufacturing technique utilizing the high capacity manufacturing process disclosed in the parent application. However, the use of the machinery is not necessarily limited to such process.

The object of the present invention, therefore, is to provide a machinery which satisfies the said need.

The invention provides, in the broadest sense thereof, an apparatus for manufacturing products based on a fluid absorbing core and having a fluid penetration preventing layer on a portion thereof, comprising means for supplying a web of a carrier material, means for supplying fluid absorbing material, for instance a defibrate of a cellulose material, onto said web, and means for folding and sealing the carrier material around the core. The apparatus is characterized by a suction device periodically operable relative the core material on the carrier material web for removing core material at locations intended as cross sealing positions of the product.

In a preferred embodiment the suction device comprises a rotatable device having suction orifices spaced from each other and adjustable rotation speed related to the desired core length.

Preferably a further suction/pressure device is arranged just downstreams of said suction device for anchoring desired core material lengths to the carrier material.

The invention will now be described with reference to the accompanying drawings, where

Figure I schematically shows a line for the manufacture of fluid absorbing products, for instance napkins, brief shields, etc.,

Figure 2 in partial view shows a strip coated carrier material web,

Figure 3 shows the web in Figure 2 cut longitudinally and together with an absorbing core,

Figure 4 shows the arrangement in Figure 3 in section,

Figure 5 in section shows the core according to Figure 4 with the carrier material applied around the core, and

Figures 6 to 9 show variants of the application of strip coated carrier material around absorbing bodies.

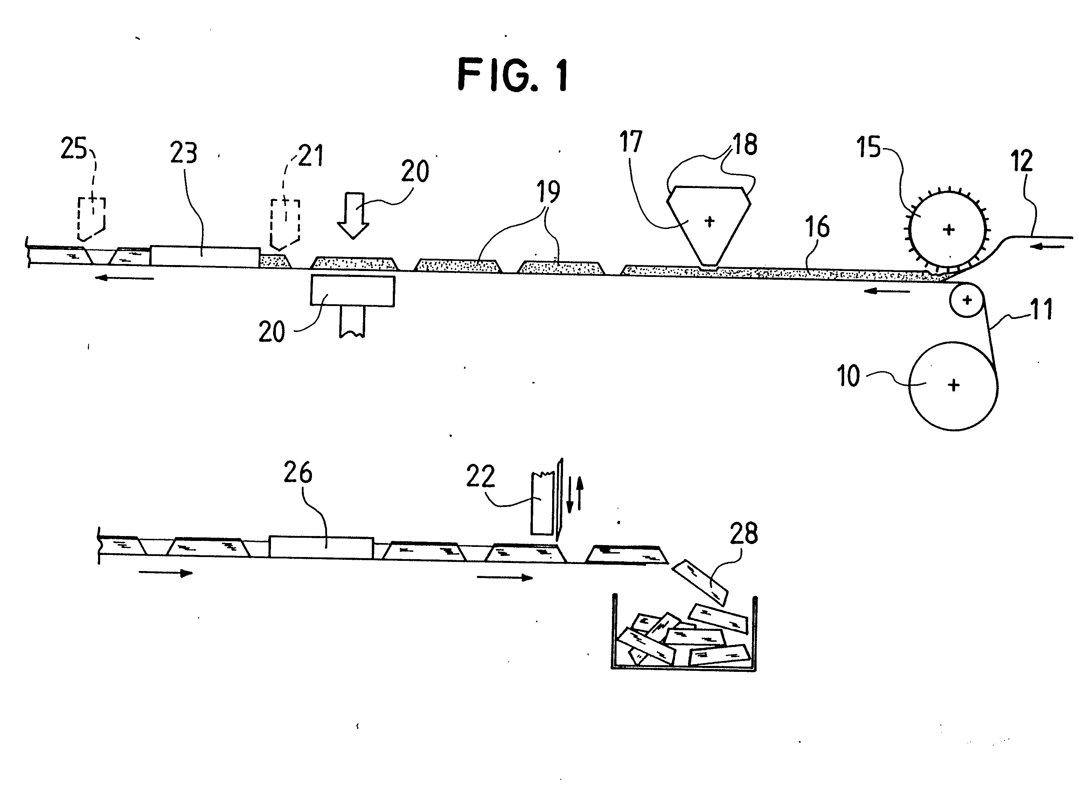

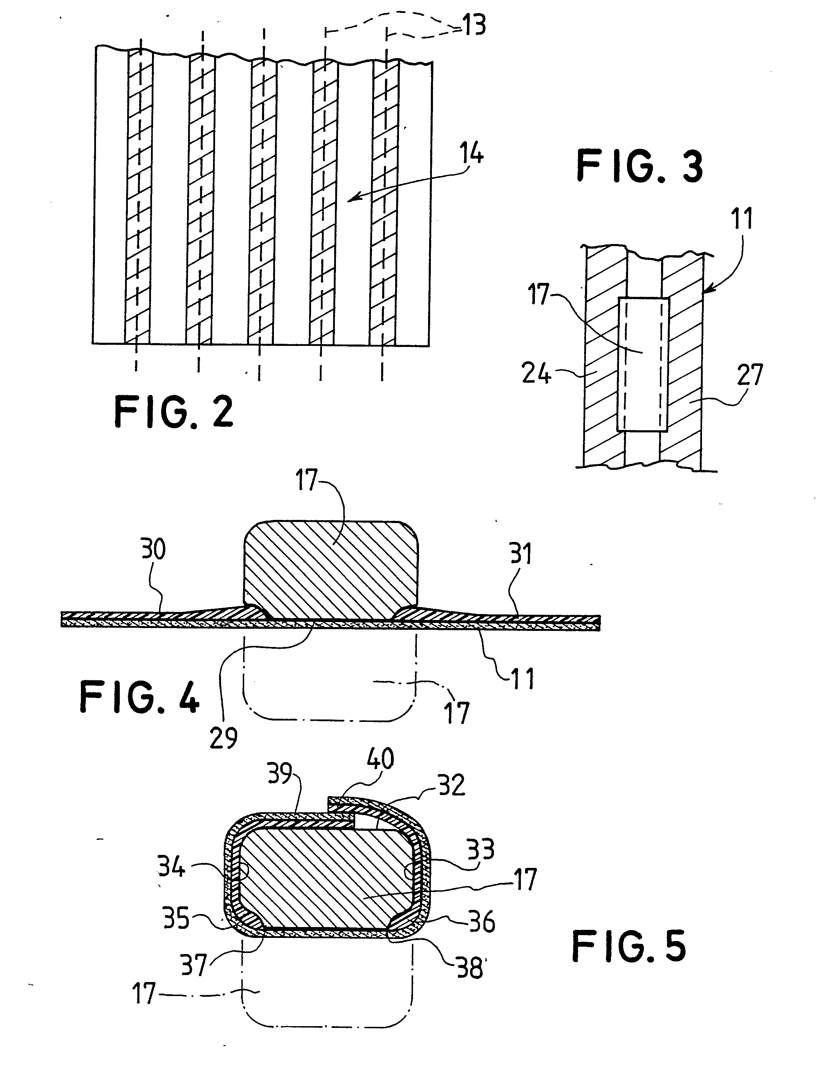

The reference numeral I0 in Figure I indicates a supply roller of carrier material. Said material consists of strip coated fibre cloth material (non-woven material) which has been extrusion strip coated by thermoplastics, for instance polyethylene, and thereafter cut length-wise into the desired width. The length-wise cutting might for instance be carried out along the broken lines I3 in Figure 2 in an extrusion strip coated, wide material web I4. This gives for instance an addition of thickness of the plastics material in strategically important positions. if the cut material is placed as for instance in Figures 3 to 5. The extrusion coating, namely, inherently implies thickening of the outer edges of the strips.

A web l2 of absorbing core material (fluff material), for instance of the defibrated cellulose type, is fed through a chopper l5 to the upper side of the web ll where the material is spread out as a string l6 of sufficient thickness for the desired absorption capacity. A cross-wise discontinuity for allowing an efficient cross-wise seal is obtained in the web l6 by a rotating multi-orifice suction device l7, in the present case comprising three suction orfices l8, one at each corner thereof. By giving the suction device a suitable rotation speed there are obtained individually separated core pieces l9 at the output side thereof.

Said pieces l9 are attached by suction to the pores of the carrier material ll in a suction/pressure station 20. The arrow in Figure l represents a pressure function and the box under the web is a suction box.

After the station 20, there eventually follows a first binding agent applicator 2l, for instance a hot melt applicator, for applying a binding agent for the cross-sealing operation in an end station 22.

A first folding bar 23, for folding one 24 (Figure 3) of the side portions of the carrier material protruding from the absorption core l7, is arranged after the suction/pressure device and applicator 2l. If required in certain cases to apply a specific binding agent in order to obtain sealing longitudinally, there is arranged a further hot melt applicator 25.

Thereafter follows a further folding station 26 for folding inwards and sealing of the other protruding edge portion of the web 27 against the already folded in first portion.

Finally, after this sealing, cross-wise sealing and cutting to finished products 28 is carried out in the end station 22.

Figures 3 to 5 show a first version of an absorbing product, for instance a sanitary towel, brief shield, etc. In this case the absorbing core l7 is placed such that one broad side 29 thereof with edge portions extended into the strip coated regions 30, 3l which here broadly coincide with the extension of the edge portions 24, 27. This implies that after the folding of the end portions to the position according to Figure 5, the coated region of the product will cover completely the upper side 32 of the core, the two longitudinal narrow sides 33, 34 and will extend around the corners 35, 36 to positions 37, 38 a distance inwards the fluid penetrating broad side 29 of the absorbing body. This has turned out to be an excellent guarantee against leakage of liquid laterally out from the body, especially as the plastics layer is thicker in the regions 37, 38. If the product for instance is a brief shield, this implies that practically all lateral leakage is prevented independently of displacing forces acting on the shield. In this connection it may be mentioned that the actual brief shield is attached to the brief in a known manner with the upper side 39 of the carrier material against the brief.

The longitudinal seal 40 shown in Figure 5 might be a dot-wise or string-wise hot melt seal, alternatively the sealing may be accomplished by directly using the thermoplastics 30, 3l.

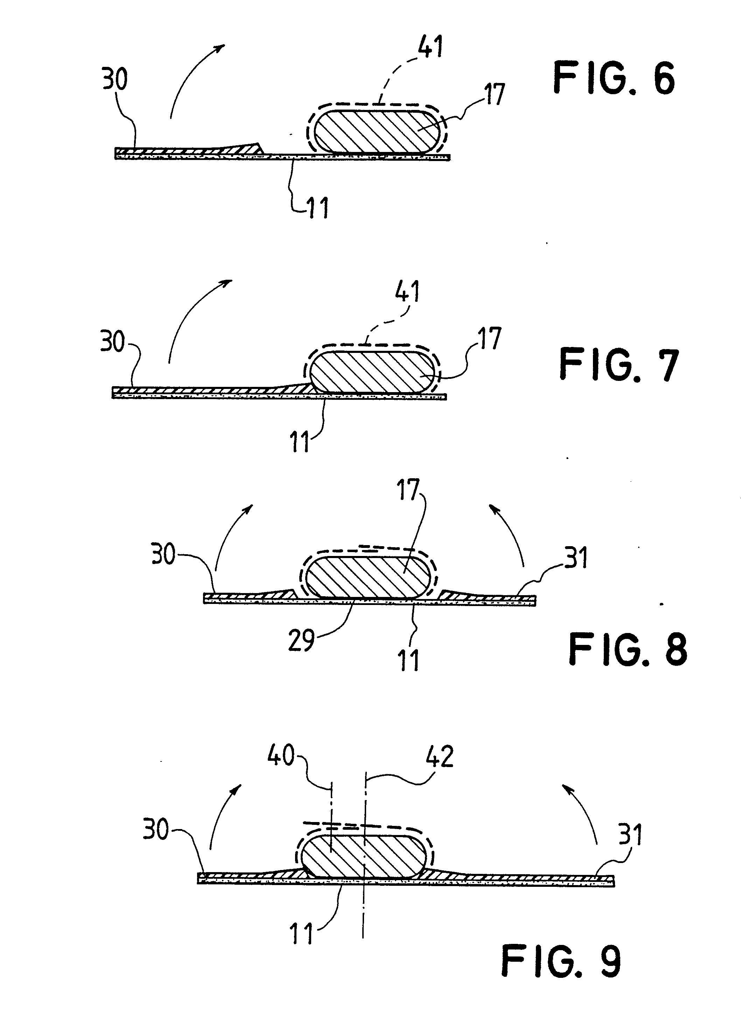

The variants in Figures 6 to 9 show different methods of longitudinally cutting the strip coated carrier material according to the invention and different placements of the absorbing body on the carrier. In the embodiment according to Figures 6 and 7 there is one strip 30 or strip portion merely and the folding of the carrier material is intended to take place along the broken lines 4l.

The version in Figure 8 discloses an embodiment where the broad side 27 of the absorbing body l7 exposed to fluid penetration is smaller than the distance between the coated strips/strip portions 30, 3l.

The embodiment according to Figure 9 resembles the one in Figure 5, but the longitudinal seal 40 is displaced relative the centre line 42 in that the strips/strip portions have different width.

If the carrier material is of the absorbing type the core l7 might advantageously be placed against the "uncoated" side of the carrier material (c.f. Figures 4 and 5).

## Claims

l. An apparatus for manufacturing products based on a fluid absorbing core and having a fluid penetration preventing layer on a portion thereof, comprising means (l0) for supplying a web (ll) of a carrier material (l2), means (l5) for supplying fluid absorbing material (l6), for instance a defibrate of a cellulose material, onto said web, and means (23, 26, 22) for folding and sealing the carrier material around the core, **characterized** by a suction device (l7) periodically operable relative the core material (l6) on the carrier material web (ll) for removing core material at locations intended as cross sealing positions of the product.

2. An apparatus as in claim l, **characterized** in that the suction device (l7) comprises a rotatable device having suction orifices (l8) spaced from each other and adjustable rotation speed related to the desired core length.

3. An apparatus as in claim 2, **characterized** in that a further suction/pressure device (20) is arranged just downstreams of said suction device (l7) and arranged for anchoring desired core material lengths to the carrier material (ll).

FIG. 1

0 231 536

FIG. 3

FIG. 2

FIG. 4

FIG. 5

FIG. 6

FIG. 7

FIG. 8

FIG. 9

**European Patent Office**

# EUROPEAN SEARCH REPORT

Application number

| DOCUMENTS CONSIDERED TO BE RELEVANT | | | EP 86202068.2 |
|---|---|---|---|
| Category | Citation of document with indication, where appropriate, of relevant passages | Relevant to claim | CLASSIFICATION OF THE APPLICATION (Int. Cl.4) |
| D,A | EP - A3 - 0 030 342 (HENKEL) <br>     * Fig. 4,5,14; claim 1 * <br> & GB-A-2 042 901 <br> ---- | 1 | A 61 F 13/16 <br> A 41 B 13/02 <br> A 61 F 5/44 |
| | | | TECHNICAL FIELDS SEARCHED (Int. Cl.4) |
| | | | A 61 F <br> A 41 B <br> A 61 L |
| | Refund: 100 % | | |

The present search report has been drawn up for all claims

| Place of search | Date of completion of the search | Examiner |
|---|---|---|
| VIENNA | 29-04-1987 | SCHÄFER |